(19) Europäisches Patentamt
European Patent Office
Office européen des brevets

(11) Numéro de publication: **0 210 893**
**B1**

(12) **FASCICULE DE BREVET EUROPEEN**

(45) Date de publication du fascicule du brevet: **03.10.90**

(21) Numéro de dépôt: **86401441.0**

(22) Date de dépôt: **30.06.86**

(51) Int. Cl.$^5$: **C 07 D 233/24,** C 07 C 255/61, A 61 K 31/415

(54) **Benzamides, leur procédé de préparation et leur application dans le domaine thérapeutique.**

(30) Priorité: **04.07.85 FR 8510199**

(43) Date de publication de la demande:
**04.02.87 Bulletin 87/06**

(45) Mention de la délivrance du brevet:
**03.10.90 Bulletin 90/40**

(84) Etats contractants désignés:
**AT BE CH DE FR GB IT LI LU NL SE**

(56) Documents cités:
**AT-A- 311 338**
**FR-A-2 248 050**
**GB-A-1 553 789**
**US-A-3 885 040**

**Le dossier contient des informations techniques présentées postérieurement au dépôt de la demande et ne figurant pas dans le présent fascicule.**

(73) Titulaire: **Laboratoires DELAGRANGE, Société Anonyme**
**1, Avenue Pierre-Brossolette**
**F-91380 Chilly-Mazarin (FR)**

(72) Inventeur: **Franceschini, Jacqueline**
**28 avenue Larroumès**
**F-94240 l'Hay-Les-Roses (FR)**
Inventeur: **Gardaix-Lutherau, Renée**
**9 rue Guichard**
**F-94230 Cachan (FR)**
Inventeur: **Margarit, Josette**
**69 avenue de Suffren**
**F-75007 Paris (FR)**

**EP 0 210 893 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

L'invention concerne de nouveaux dérivés de benzamide et leurs sels pharmacologiquement acceptables, répondant à la formule générale (I):

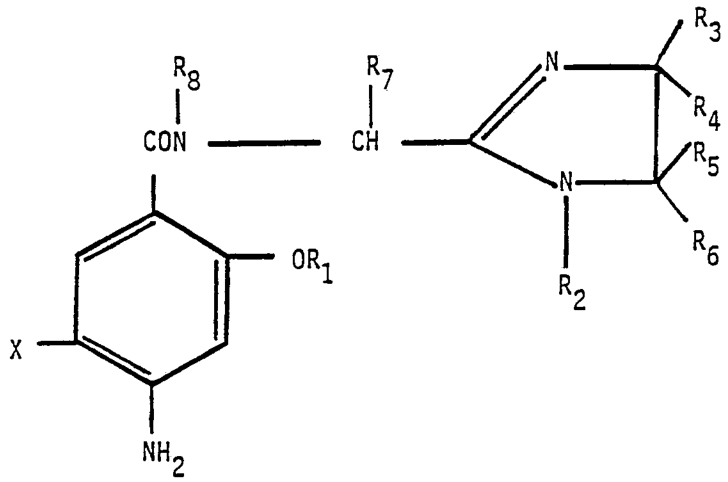

(I)

dans laquelle:

$R_1$ représente un atome d'hydrogène, un radical méthyle ou allyle.

$R_2$ représente un atome d'hydrogène, un radical méthyle ou éthyle, allyle, benzyle, cyclopropylméthyle ou cyclo-hexénylméthyle,

$R_3$, $R_4$, $R_5$, $R_6$, $R_7$ et $R_8$ représentent des atomes d'hydrogène ou des radicaux méthyle.

X représente un atome de chlore ou de brome,

On connaît des dérivés de 4-amino 5-halo ortho-anisamide et en particulier le N-(diéthylaminoéthyl) 2-méthoxy 4-amino 5- chloro benzamide (métoclopramide), comme agents antiémétiques et modificateurs du comportement digestif.

Le métoclopramide a fait l'objet de nombreux brevets et publications décrivant ses méthodes de préparation et ses propriétés thérapeutiques. Des études détaillées de son activité sur le système gastro-intestinal et de ses effets secndaires ont été publiées par exemple dans Gastroenterology, *77*, (1979), 768—769 et Drugs *25* (1983), 451—494.

Aucun dérivé de N-(2-imidazolinylméthyl) benzamide, ayant ou non un effet gastromoteur, n-étant connu, le métoclopramide, en raison de la structure de son noyau et de ses propriétés, peut être considéré comme le composé de l'art connu le plus proche des composés de l'invention et a donc été utilisé comme composé de référence dans la présente demande de brevet.

Le métoclopramide, comme la plupart des composés de structure analogue, a une activité antidopaminergique importante, ce qui a l'inconvenient de provoquer des troubles neurologiques.

Contrairement à ce qui pouvait être attendu, on a découvert que les composés de formule (I) telle que définie ci-dessus, tout en étant doués de propriétés gastromotrices équivalentes à celles du métoclopramide, agissaient peu sur les récepteurs dopaminergiques centraux et étaient donc dépourvus des effets secondaires indésirables fréquents dans la série des benzamides de structure voisine.

Les composés selon l'invention peuvent être préparés par réaction d'un acide de formule générale (II):

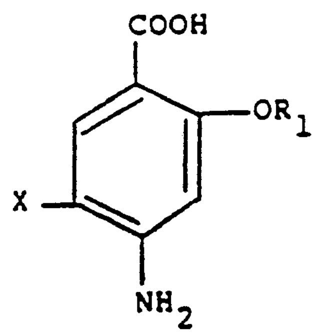

(II)

dans laquelle $R_1$ et X ont les mémes significations que précédemment, avec un nitrile de formule générale (III):

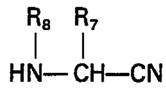

(III)

pour obtenir un dérivé de cyanométhylbenzamide substitué de formule générale (IV):

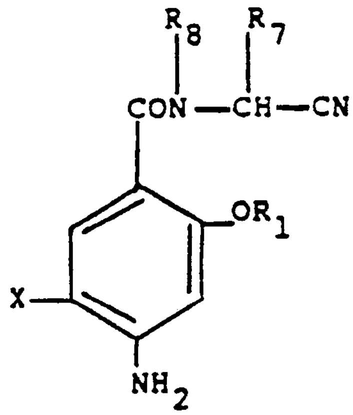

(IV)

que réagira avec une diamine substituée pour donner le composé de formule (I) attendu.

Selon une variante de ce procédé de synthèse, on peut préparer à partir d'un acide de formule (II) un dérivé réactif de cet acide, tel que par exemple un ester mixte de formule générale (V)

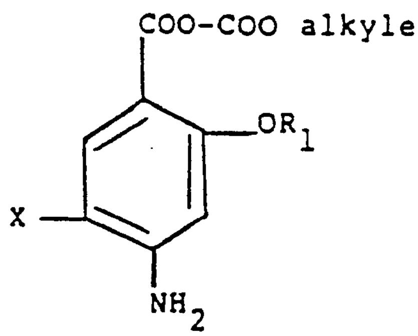

(V)

ou un chlorure d'acide de formule générale (VI)

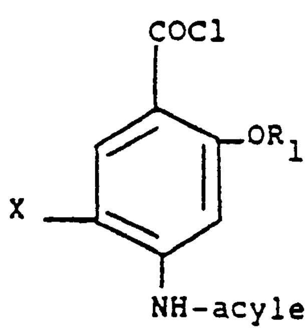

(VI)

dans lesquels les valeurs de $R_1$ et de X sont les mêmes que précédemment défini, que réagira sur un nitrile de formule (III) pour donner le dérivé de cyanométhylbenzamide de formule (IV) et enfin le composé de formule (I) attendu.

Certains des intermédiaires de synthèse sont des composés nouveaux, en particulier, les nitriles de formule (IV) et les esters mixtes de formule (V).

Afin d'illustrer l'invention, des exemples vont être décrits.

Exemple I

N-[(1-ethyl 2-imidazolin 2-yl)methyl] 2-methoxy 4-amino 5-chloro benzamide

Stade 1: N(cyanomethyl) 2-methoxy 4-amino 5-chloro benzamide

Dans un ballon de trois litres muni d'un agitateur étanche, d'un réfrigérant à reflux, d'un thermomètre et d'une ampoule à brome, on introduit 100 g d'acide 2-méthoxy 4-amino 5-chloro benzoïque (0,5 mole), 655 cc de chloroforme séché sur $Cl_2Ca$, et 51 g de triéthylamine séchée sur potasse (0,5 mole). Après dissolution de l'acide, on observe la précipitation immédiate du sel formé. On refroidit à 0°—5° la suspension obtenue et on coule goutte à goutte 54 g de chloroformiate d'éthyle (0,5 mole). Le précipité se dissout au fur et à mesure de la coulée et en fin d'addition on obtient une solution que l'on agite encore pendant 1 heure à 0°.

On ajoute alors goutte à goutte une solution de 51 g de chlorhydrate d'aminoacétonitrile (0,5 mole + 10% d'excès) dans 150 cc de chloroforme et 56 g de triéthylamine séchée sur potasse (0,5 mole + 10% d'excès) en maintenant la température entre 5° et 10° : il y a formation d'un précipité.

L'addition terminée on agite encore pendant une heure à 10°.

On élimine ensuite la plus grande partie du chloroforme, on reprend le résidu à l'eau et alcalinise par addition de lessive de soude à 30%. Le précipité restant est essoré, lavé à l'eau jusqu'à neutralité des eaux de lavage et séché à 40° en étuve ventilée.

Poids obtenu = 106 g   Rendement = 88,5%

F = 196°C

Stade 2: N[(1-ethyl 2-imidazolin 2-yl) methyl] 2-methoxy 4-amino 5-chloro benzamide

Dans un ballon de 250 cc mini d'un agitateur étanche, d'un réfrigérant à reflux et d'un thermomètre, on introduit 26 g de N (cyanométhyl) 2-méthoxy 4-amino 5-chloro benzamide (0,109 mole), 19 g de N-éthyl-éthylènediamine (2 × 0,109 mole) et 5 gouttes de sulfure de carbone. On obtient une épaisse bouillie difficilement agitable que l'on chauffe au bain d'huile. A 110° on obtient une solution et l'ammoniac formé se dégage. On continue de chauffer jusqu'à 120°—130°. Au bout de 12 minutes, le dégagement gazeux est terminé. On enlève alors le bain d'huile et on refroidit en agitant jusque vers 40°. On ajoute alors 200 cc d'eau glacée. Le précipité qui se forme est essoré, lavé à l'eau et séché à 40° en étuve ventilée.

Poids obtenu = 25 g   Rendement = 74%

Le produit obtenu contient un peu de nitrile de départ. Il est redissous dans l'eau et l'acide acétique nécessaire. L'insoluble est filtré et la solution est alcalinisée par addition de lessive de soude à 30% jusqu'à virage de la phénolphtaléine. Le produit qui précipite est essoré, lavé à l'eau jusqu'à neutralité des eaux de lavage et séché à 40°.

Poids obtenu = 20 g   Rendement de la purification = 80%

Le composé est obtenu sous forme de cristaux blancs, solubles dans l'acide acétique dilué, et très soluble dans le chloroforme (F = 175°—176°, Büchi).

Exemple II

N-[(1)Allyl 2-imidazolin -2-yl) methyl] 2-methoxy 4-amino 5-chloro benzamide

Dans un ballon de 500 cc mini d'un agitateur étanche, d'un réfrigérant à reflux surmonté d'un compte-bulles contenant de l'huile de vaseline, d'un thermomètre et d'une ampoule à brome, on introduit 54 g de N(cyanométhyl) 2-méthoxy 4-amino 5-chloro benzamide finement pulvérisés, et 45 g de N-allyl éthylènediamine. On obtient une bouillie très épaisse difficilement agitable que l'on chauffe à 100° à l'aide d'un bain d'huile. On ajoute alors 15 gouttes de sulfure de carbone. On observe une élévation rapide de la température à 110° et un dégagement gazeux très important. En même temps la bouillie initiale se dissout lentement. Au bout de 10 minutes, le dégagement gazeux ralentissant, on ajoute de nouveau 12 gouttes de sulfure de carbone. Le dégagement d'ammoniac reprend, le produit initial se dissout entièrement puis le dérivé formé cristallise. La température s'élève jusqu'à 135°. Cinq minutes après, le dégement gazeux est terminé. On refroidit immédiatement jusque vers 15°—20° et on reprend le mélange réactionnel par 1 litre d'eau. Le produit obtenu est essoré, lavé abondamment à l'eau et séché à 40° en étuve ventilée.

Poids obtenu = 63 g   Rendement = 87%

Ces 63 g de produit sont redissous à chaud dans 126 cc de 2-méthoxy éthanol. La solution bouillante est filtrée avec du noir puis refroidie. La base qui recristallise est essorée, lavée avec du 2-méthoxy éthanol et séchée à 40°.

Poids obtenu = 46,5 g   Rendement = 74%
Rendement total = 64%

F = 177°—178°C.

Exemple III

Chlorhydrate de n-[(1-benzyl 2-imidazolin 2-yl) methyl] 2-methoxy 4-amino 5-chloro benzamide

Stade 1: N-[(1-benzyl 2-imidazolin 2-yl) methyl)] 2-methoxy 4-amino 5-chloro benzamide

Dans un ballon de 500 cc mini d'un agitateur étanche, d'un réfrigérant à reflux surmonté d'un compte-bulles contenant de l'huile de vaseline, d'un thermomètre et d'un ampoule à brome, on introduit 81.5 g de N-(cyanométhyl) 2-méthoxy 4-amino 5-chlor benzamide et 102 g de N-benzyl éthylènediamine et on chauffe jusqu'à 105°. On coule alors 15 gouttes de sulfure de carbone. La suspension initiale se fluidifie rapidement. La température s'élève à 125° et en même temps il se produit un dégagement gazeux important.

Au bout de 10 minutes, le dégagement gazeux ralentissant, on ajoute de nouveau 15 gouttes de sulfure de carbone. Le dégegement reprend aussitôt, la température s'élève à 129° et tout se dissout. Le dégagement gazeux teminé, on arrête la réaction et on refroidit. Le produit qui cristallise est repris immédiatement par 500 cc d'éther, essoré, lavé à l'éther, séché à l'air puis à 40°.

Poids obtenu = 125 g  Rendement = 98%

Ces 125 g de produit sont recristallisés dans 125 cc de 2-méthoxy éthanol.

Poids obtenu = 84 g  Rendement de cristallisation = 67%

Ces 84 g de base sont redissous dans l'eau et l'acide acétique nécessaire pour ajuster le pH à 4. La solution obtenue est filtrée avec du noir puis alcalinisée par addition d'ammoniaque à 20% jusqu'à virage de la phénolphtaléine. La base qui précipite est essorée, lavée à l'eau jusqu'à neutralité des eaux de lavage et séchée à 40°.

Poids obtenu = 61 g  Rendement de la purification = 73%

F = 165°—156°

La base obtenue reste colorée et contient toujours une légère impureté. Elle est recristallisée une nouvelle fois dans 61 cc de 2-éthoxy éthanol. Après essorage, elle est lavée avec du 2-éthoxy éthanol glacé puis avec de l'eau pour éliminer toute trace de solvant et elle est séchée à 40°.

Poids obtenu = 57,5 g  Rendement de cristallisation = 94%
Rendement des purifications = 46%
Rendement total = 45%

Stade 2: Chlorhydrate de n-[(1-benzyl 2-imidazolin 2-yl) methyl] 2-methoxy 4-amino 5-chloro benzamide

57,5 g de base sont dissous à chaud dans 230 cc d'éthanol absolu et une solution de 5,7 g de gaz chlorhydrique dans 15 cc d'éthanol est ajoutée jusqu'à virage du rouge congo.

La solution obtenue est alors refroidie. Le chlorhydrate formé cristallise lentement.

Il est essoré au bout de plusieurs heures, lavé à l'éthanol absolu et séché à 40°.

Poids obtenu: le jet =60 g  P.M. ($AgNO_3$ N) = 413
2ème jet = 3 g

Poids total obtenu 63 g  Rendement = 100%

Ces 63 g de chlorhydrate sont recristallisés dans 190 cc d'éthanol absolu.

Poids obtenu = 54 g  Rendement de recristallisation = 86%

Le chlorhydrate obtenu retient des traces d'éthanol qui ne disparaissent pas même après séchage prolongé sous vide à 45° puis 70°.

Finalement le produit est repris rapidement à l'eau, essoré et séché.

Poids obtenu = 40 g  Rendement de la purification = 74%

P.M. ($AgNO_3$ $\frac{N}{10}$) = 410,5

F = 258°C  Rendement total: 63%

Exemple IV

N[(1-cyclopropylmethyl 2-imidazolin 2-yl) methyl] 2-methoxy 4-amino 5-chloro benzamide

Dans un ballon de 500 cc muni d'un agitateur étanche, d'un réfrigérant à reflux surmonté d'un compte-bulles contenant de l'huile de vaseline, d'un thermomètre et d'une ampoule à brome, on introduit 45 g de N-(cyanométhyl) 2-méthoxy 4-amino 5-chloro benzamide finement pulvérisés et 43 g de N-cyclopylméthyl éthylène diamine. On obtient une épaisse bouillie que l'on chauffe au bain d'huile à 110°. On ajoute alors peu à peu 20 gouttes de sulfure de carbone. On observe immédiatement un important dégagement d'ammoniac et une élévation rapide de la température jusqu'à 120°. En même temps le milieu se fluidifie et au bout de 6 minutes tout se dissout.

Peu après, le produit formé cristallise. Puis la température redescend et le dégagement gazeux diminue nettement avant de s'arrêter complètement.

Après refroidissement le précipité est repris à l'éther. il est ensuite essoré, lavé à l'éther et séché à l'air puis à 50°.

Poids obtenu = 64 g Rendement = 100%

Ces 64 g de produit sont redissous à chaud dans 225 cc d'éthanol absolu. La solution bouillante est filtrée avec du noir puis refroidie. La base qui recristallise alors rapidement est essorée, lavée à l'éthanol puis à l'éther, séchée à l'air puis à 50°.
Poids obtenu = 29 g Rendement de recristallisation = 45,5%
F = 184°C.

Un deuxième jet permet de récupérer 11 g de produit. Ces 11 g de produit sont recristallisés aussitôt dans 33 cc d'éthanol absolu.
Poids obtenu = 7 g
Poids total obtenu: 36 g. Rendement total de recristallisation = 56%

Ces 36 g de base sont recristallisés dans 125 cc d'éthanol absolu puis les 29 g de produit obtenus sont recristallisés dans 145 cc d'isopropanol.
Poids obtenu = 27,5 g Rendement de recristallisation = 76,5%
F = 182,5—184,5°C Rendement total = 43%

Exemple V

N-[(1-ethyl 2-imidazolin 2-yl) methyl] 2-allyloxy 4-amino 5-chloro benzamide

Stade 1: 2-Hydroxy 4-acetamino 5-chloro benzoate d'ethyle

Dans un ballon de 3 litres mini l'un agitateur étanche, d'un réfrigérant à reflux et d'un thermomètre, on introduit 258 g de 2-hydroxy 4-acétamino benzoate d'éthyle et 1150 cc d'acide acétique et on chauffe au bain d'eau à 40° jusqu'à dissolution. On ajoute alors 153,5 g de N-chlorosuccinimide et on continue de chauffer à 40°—50° jusqu'à dissolution totale. On abandonne ensuite la solution obtenue dans une étuve à 50° pendant 48 heures. On obtient alors un produit cristallisé qui est repris par 8 litres d'eau. Ces cristaux sont essorés, lavés abondamment à l'eau jusqu'à neutralité des eaux de lavage et séchés à 40° en étuve ventilée.
Poids obtenu = 226 g Rendement = 89%
F = 150°C

Ces 266 g sont recristallisés dans 300 cc d'acide acétique.

Poids obtenu = 198 g Rendement de recristallisation = 74,5%
F = 159°C Rendement total = 66%

Stade 2: 2-Allyloxy 4-acetamino 5-chloro benzoate d'éthyle

Dans un ballon d'un litre muni d'un agitateur étanche, d'un réfrigérant à reflux et d'un thermomètre, on introduit 53 g de carbonate de potassium pulvérisés, 5,5 g de chlorure de benzyl tributylammonium, 220 cc d'acétonitrile et 46,5 g de bromure d'allyle puis 99 g de 2-hydroxy 4-acétamino 5-chloro benzoate d'éthyle. On chauffe au reflux la suspension obtenue, pendant 3 heures 30 jusqu'à ce qu'une prise d'essai de solution claire diluée dans un peu d'alcool ne donne aucune coloration avec le chlorure ferrique. On distille alors une partie de l'acétonitrile et on reprend le résidu à l'eau. Les sels minéraux se dissolvent. L'ester allyloxy qui précipite est essoré, lavé à l'eau jusqu'à élimination des ions $Br^-$ et séché à 35° en étuve ventilée.
Poids obtenu = 130 g Théorie = 114 g

Le produit obtenu est à moitié cristallisé. Il est redissous dans 260 cc d'éthanol à 60% bouillant. La solution obtenue est trouble. Elle est filtrée avec du noir puis refroidie. L'ester qui recristallise est essoré, lavé à l'éthanol à 60% et séché en étuve ventilée à 40°.
Poids obtenu = 70,5 g Rendement de recristallisation = 62%
F = 80°C Rendement total = 62%

Stade 3: Acide 2-allyloxy 4-amino 5-chloro benzoique

Dans un ballon d'un litre mini d'un réfrigérant à reflux, on introduit 68 g de 2-allyloxy 4-acétamino 4-chloro benzoate d'éthyle, 172 cc d'êthanol et 50,5 cc de lessive de soude à 30% et on chauffe au reflux au bain-marie pendant 3 heures. On reprend alors la solution obtenue par 2 litres d'eau, on la filtre avec du noir pour e'liminer un trouble puis on l'acidifie par addition de 45 cc d'acide chlorhydrique concentré (virage du rouge congo). L'acide qui précipite est essoré après refroidissement, lavé à l'eau jusqu'à neutralité des eaux de lavage et séché à 50°.
Poids obtenu = 50 g Rendement = 95%
P.M. (KOH $\frac{N}{10}$—potentiométrie) = 232
F = 142°C

Stade 4: N-(cyanomethyl) 2-allyloxy 4-amino 5-chloro benzamide

Dans un ballon de 500 cc muni d'un agitateur étanche, d'un réfrigérant à reflux, d'un thermomètre et d'une ampoule à brome, on introduit 51 g d'acide 2-allyloxy 4-amino 5-chloro benzoïque, 220 cc de chloroforme et 23 g de triéthylamine. On refroidit la solution obtenue à 0°: le sel de triéthylammonium cristallise. On ajoute alors goutte à goutte, à cette suspension, 24,5 g de chloroformiate d'éthyle en maintenant la température entre 0° et 5° par refroidissement dans un bain de glace. Le précipité se dissout peu à peu et finalement on obtient une solution que l'on agite encore pendant 30 minutes. On y ajoute alors une solution de 21 g de chlorhydrate d'aminoacétonitrile dans 220 cc de chloroforme et 23 g de triéthylamine. La température s'élève jusqu'à 32°. L'addition terminée, on continue d'agiter encore pendant 1 heure. On élimine alors la plus grande partie du chlorforme et on reprend le résidu à l'eau. le produit obtenu est essoré, lavé à l'eau, à la soude diluée, puis de nouveau à l'eau et séché à 40° en étuve ventilée.

Poids obtenu = 49,5 g Rendement = 83%
F = 149°C

Stade 5: N-[(1-ethyl 2-imidazolin 2-yl) methyl] 2-allyloxy 4-amino 5-chloro benzamide

Dans un ballon de 500 cc mini d'un agitateur étanche, d'un réfrigérant à reflux surmonté d'un tube compte-bulles contenant de l'huile de vaseline, d'un thermomètre et d'une ampoule à brome, on introduit 49 g de N(cyanométhyl) 2-allyloxy 4-amino 5-chloro benzamide et 32,5 g de N-éthyléthylènediamine et on chauffe à 120° à l'aide d'un bain d'huile préalablement chauffé à 120°. On obtient aussitôt une solution. On ajoute alors 10 gouttes de sulfure de carbone. On observe immédiatement la formation de vapeurs blanchâtres à l'intérieur du ballon et un dégagement gazeux.

On continue de chauffer à 120° jusqu'à la fin du dégement gazeux, soit 1 heur 30. On refroidit alors à 40° et on ajoute du chloroforme. On lave à l'eau la solution chloroformique obtenue de mainière à éliminer l'amine en excès. On la sèche ensuite sur sulfate de sodium. On évapore le chloroforme sous vide léger et on reprend le résidu à l'acétate d'éthyle. Le produit qui recristallise est essoré, lavé à l'acétate d'éthyle et séché à 40°.

Poids obtenu = 39 g Rendement = 63%

Ces 39 g de produit sont redissous à chaud dans 75 cc de méthyléthylcétone. La solution bouillante est filtrée avec du noir puis refroidie. La base qui cristallise est essorée, lavée à la méthyléthylcétone et séchée à 40°.

Poids obtenu = 31 g Rendement de recristallisation = 80%

On recristallise ces 31 g de base dans 65 cc d'isopropanol. Après essorage, le produit recristallisé est lavé à l'isopropanol puis à l'éther. Il est ensuite séché à l'air puis à 40°.

Poids obtenu = 27 g Rendement de recristallisation = 87%
F = 136,5 — 137,5°C Rendement des recristallisations = 70%
Rendement total = 44%

## Exemple VI

N-[(1-ethyl 2-imidazolin 2-yl) methyl] 2-methoxy 4-amino 5-bromo benzamide

Stade 1: N-(cyanomethyl) 2-methoxy 4-amino 5-bromo benzamide

Dans un ballon de deux litres mini d'un agitateur étanche, d'un réfrigérant à reflux, d'un thermomètre et d'une ampoule à brome, on introduit 107 g d'acide 2-méthoxy 4-amino 5-bromo benzoïque finement pulvérisés, 531 cc de chloroforme et 44 g de triéthylamine, et on chauffe jusqu'à dissolution. On refroidit ensuite à 0° et on coule goutte à goutte dans la solution obtenue 47 g de chloroformiate d'éthyle en maintenant la température entre 0° et 5° par refroidissement. L'addition terminée, on agite encore pendant 1 heur entre 0° et 5°. Simultanément, dans un ballon d'un litre mini d'un agitateur, on introduit 44 g de chlorhydrate d'aminoacétonitrile, 531 cc de chloroforme et après refroidissement à 0°, on ajoute 48 g de triéthylamine. Il se forme alors un précipité très fin. Cette suspension est ajoutée peu à peu à la solution chloroformique d'anhydride mixte obtenue précédemment, en maintenant la température entre 0° et 5°. L'introduction terminée, on agite encore pendant 1 heure entre 0° et 5° puis on laisse remonter la température.

On obtient alors un précipité gris, gélatineux en suspension dans le chloroforme. On ajoute 1 litre d'eau et on entraîne la totalité du chloroforme par distillation.

Puis on refroidit et on ajoute 10 cc de lessive de soude à 10% de manière à redissoudre des traces d'acide n'ayant pas réagi. Le produit restant est alors essoré, lavé à l'eau jusqu'à élimination des ions $Cl^-$ et séché à 50°.

Poids obtenu = 117 g Rendement = 95%
F = 200°C

Le produit est remis en suspension dans un litre d'eau et 10 cc de lessive de soude à 10%, essoré, lavé à l'eau jusqu'à neutralité des eaux de lavage et séché à 50°.

Poids obtenu = 108 g Rendement de purification = 92%
F = 206°C Rendement total = 88%

Stade 2: N-[(1-ethyl 2-imidazolin 2-yl) methyl] 2-methoxy 4-amino 5-bromo benzamide

Dans un ballon de 500 cc muni d'un agitateur étanche, d'un réfrigérant à reflux surmonté d'un compte-bulles contenant de l'huile de vaseline, d'un thermomètre et d'une ampoule à brome, on introduit 85 g de N-(cyanométhyl) 2-méthoxy 4-amino 5-bromo benzamide finement pulvérisés et 53 g de N-éthyl éthylènediamine et on chauffe au bain d'huile jusqu'à 105° l'épaisse suspension obtenue. On ajoute alors 20 gouttes de sulfure de carbone. Immédiatement la réaction démarre avec dégagement d'ammoniac. Le milieu se fluidifie et se transforme rapidement en un liquide épais. Peu après, le produit formé cristallise. On refrodit aussitôt et reprend à l'éther.

Le produit obtenu est essoré, lavé à l'éther et séché à l'air.

Poids obtenu = 102 g  Rendement = 96%

Ces 102 g sont redissous dans 200 cc de chloroforme. Il reste un léger insoluble qui est filtré sous vide. Puis le chloroforme est totalement éliminé. Le résidu qui cristallise est aussitôt redissous dans 153 cc d'éthanol bouillant. La solution obtenue est refroidie jusque vers 10°.

La base qui recristallise est essorée, lavée à l'éthanol glacé puis à l'éther, séchée à l'air puis à 50° et enfin sous vide à 50°.

Poids obtenu: ler jet = 43 g  F = 178°C
2ème jet = 24 g  F = 178°C

Poids total obtenu = 67 g  Rendement de recristallisation = 66%

P.M. ($ClO_4H$ $\frac{N}{10}$) = 355,5

F = 176,5 — 178,5°C  Rendement total = 63%

Exemple VII

Methane sulfonate de n-methyl n-[(1-ethyl 2-imidazolin 2-yl) methyl] 2-methoxy 4-amino 5-chloro benzamide

Stade 1: N-methyl n-(cyanomethyl) 2-methoxy 4-amino 5-chloro benzamide

Dans un ballon de deux litres muni d'un agitateur étanche, d'un réfrigérant à reflux, d'un thermomètre et d'une ampoule à brome, on introduit 101 g d'acide 2-méthoxy 4-amino 5-chloro benzoïque, 250 cc de chloroforme et 50,5 g de triéthylamine. On chauffe jusqu'à dissolution, puis on refroidit à 0°: le sel de triéthylammonium formé cristallise. On coule alors goutte à goutte dans la solution obtenue 54,5 g de chloroformiate d'éthyle en maintenant la température entre 0° et 5°. L'addition terminée, on agite encore 30 minutes à 5°. Simultanément dans un ballon d'un litre mini d'un agitateur, on introduit 400 cc de chloroforme et 55,5 g de triéthylamine et on refroidit le tout dans un bain de glace. On ajoute alors peu à peu 59 g de chlorhydrate de méthylamino acégtonitrile finement pulvérisés.

La solution obtenue est ensuite ajoutée peu à peu à la solution d'anhydride mixte obtenue précédemment, en maintenant la température entre 5° et 10°. En fin d'addition on agite encore pendant 1 heure à 5° puis on laisse remonter la température et on abandonne le milieu réactionnel une nuit.

Le chloroforme est ensuite distillé sous léger vide. Le résidu est repris à l'eau et les dernières traces de chloroforme sont enlévées par entraînement à l'eau. Le produit restant cristallise.

On ajoute alors quelques gouttes de lessive de soude pour alcaliniser légèrement le milieu puis on essore le produit obtenu, on le lave à l'eau jusqu'à élimination des ions $Cl^-$ et on le sèche à 40°.

Poids obtenu = 92 g  Rendement = 72,5%

F = 128°C

Stade 2: N-methyl N-[(1-ethyl 2-imidazolin-2-yl) methyl] 2-methoxy 4-amino 5-chloro benzamide

Dans un ballon de 250 cc mini d'un agitateur étanche, d'un réfrigérant à reflux, d'un thermomètre et d'une ampoule à brome, on introduit 50 g de N-méthyl N-(cyanométhyl) 2-méthoxy 4-amino 5-chloro benzamide finement pulvérisés et on chauffe jusque vers 80°. On ajoute 35 g de N-éthyl éthylènediamine et on chauffe jusqu'à 120° la solution obtenue. On ajoute alors 5 gouttes de sulfure de carbone et on maintient le chauffage entre 125° et 130° jusqu'à la fin du dégagement d'ammoniac, soit 2 heures. La solution est alors reprise à l'eau glacée et elle est acidifiée par addition d'acide acétique. Elle est aussitôt filtrée avec du noir puis alcalinisée, en refroidissant bien, par un large excès de lessive de soude à 30%. L'huile qui se sépare est décantée et extraite trois fois au chlorforme. La solution chloroformique est ensuite lavée deux fois à l'eau puis séchée sur sulfate de sodium. Puis le chloroforme est distillé en terminant sous vide jusqu'à poids constant.

Poids obtenu = 73 g  Théorie = 64 g

Le produit ainsi obtenu contient 25% de chloroforme ce qui représente 55 g de produit correct soit un rendement de 86%.

Purification:

129 g de produit (correspondant à 96% de base pure) sont dissous à chaud dans 200 cc d'éthanol et 26 g d'acide oxalique. Après refroidissement, l'oxalate précipite en une masse gélatineuse qui finit par se solidifier.

Le produit obtenu est essoré, lavé à l'éthanol et séché à 40°.

Poids obtenu = 110 g Rendement = 91,5%

Les 110 g d'oxalate obtenus sont dissous dans 1 litre d'eau. Il reste alors un insoluble qui est filtré avec du noir. La solution obtenue est ensuite alcalinisée en refroidissant extérieurement dans un bain glacé, par addition de 150 cc de lessive de soude à 30%.

L'huile qui se sépare est décantée et extraite au chloroforme. La solution chloroformique est lavée deux fois à l'eau puis séchée sur sulfate de sodium. Le chloroforme est ensuite distillé sous vide jusqu'à poids constant.

Poids obtenu = 93 g

Le produit obtenu contient encore 19 à 20% de chloroforme ce qui donne un poids réel de base de 75 g.

Poids réel de base = 75g Rendement de la purification = 78%

Stade 3: Methane sulfonate de n-methyl n-[(1-ethyl 2-imidazolin-2-yl) methyl] 2-methoxy 4-amino 5-chloro benzamide

93 g de produit (correspondant à 75 g de base pure) sont dissous dans 220 cc d'isopropanol et 22,5 g d'acide méthane sulfonique. La cristallisation est amorcée. Le méthane sulfonate formé cristallise alors lentement. Il est abandonné pendant une nuit puis essoré, lavé à l'isopropanol et séché à 40°.

Poids obtenu = 74 g Rendement = 76%

Ces 74 g de méthane sulfonate sont recristallisés dans 185 cc d'isopropanol. Après essorage, lavage et séchage, on récupère:

Poids obtenu = 70 g Rendement de la recristallisation = 95%

Le produit ainsi obtenu retient de l'isopropanol qui n'est pas éliminé par séchange prolongé à 40° sous pression réduite. Il est de ce fait redissous dans 45 cc d'eau. La solution est transvasée dans un grand cristallisoir et le tout est abandonné d'abord à l'air puis à l'étuve ventilée à 20° pour évaporer le maximum d'eau. Le produit restant est ensuite séché à fond à 40°.

| | |
|---|---|
| Poids obtenu = 67 g | Rendement de la purification = 96% |
| P.M. ($Cl^-$ après minéralisation) = 421 | Rendement des purifications = 91% |
| F = 163,5—164°C | Rendement total de la réaction = 69% |

Exemple VIII

Chlorhydrate de N-[(1-methyl 2-imidazolin-2-yl) methyl] 2-methoxy 4-amino 5-chloro benzamide

Stade I: N-[(1-methyl 2-imidazolin-2-yl) methyl] 2-methoxy 4-amino 5-chloro benzamide.

Dans un ballon de 250 cc muni d'un agitateur étanche, d'un réfrigérant à reflux surmonté d'un compte-bulles contenant de l'huile de vaseline, d'un thermomètre et d'une ampoule à brome, on introduit 48 g de N-(cyanométhyl) 2-méhtoxy 4-amino 5-chloro benzamide et 30 g de N-méthyl éthylène diamine.

On chauffe à 105° au bain d'huile la suspension obtenue et on ajoute alors 15 gouttes de sulfure de carbone. Il se produit immédiatement un dégagement d'ammoniac et la suspension initiale se dissout. On maintient le chauffage à 105° jusqu'à la fin du dégagement gazeux, soit 25 minutes et on refroidit.

Le produit qui commence à cristalliser est repris à l'éther, essoré, lavé à l'éther puis à l'éthanol et séché.

Poids obtenu = 45 g Rendement = 76%

F = 228°C

Ces 45 g de produit sont recristllisés dans 180 cc de 2-méthoxy éthanol. Le produit qui recristallise en refroidissant est essoré, lavé avec du 2-méthoxy éthanol et séché à 50°.

Poids obtenu = 36,5 g Rendement de recristallisation = 81%

P.M. ($HCl\,O_4$ $\frac{N}{10}$ — potentiométrie) = 229

F (Büchi) = 216—219°C.

Stade 2: Chlorhydrate de N-[(1-Methyl 2-imidazolin-2-yl) methyl] 2-methoxy 4-amino 5-chloro benzamide

A 45 g de base finement pulvérisés en suspension dans 90 cc d'éthanol, on ajoute une solution de 5,5 g de gaz chlorhydrique dans 8 cc d'éthanol absolu. Après dissolution de la base, le chlorhydrate formé cristallise. Il est essoré, lavé à l'éthanol absolu, séché à 50° puis sous vide à 40° pour enlever toute trace de solvant.

Poids obtenu = 43 g
P.M. ($AgNO_3$ $\frac{N}{10}$) = 344 } Ce qui correspond à un produit contenant environ 1/2 mole d'eau
$H_2O$ (Fischer) = 3%
F = 207,5—210°C    Rendement en produit hydraté = 84%

Exemple IX

N-[(1-ethyl 4-methyl 2-imidazolin-2-yl methyl] 2-methoxy 4-amino 5-chloro benzamide

Dans un ballon de 250 cc muni d'un agitateur étanche, d'un réfrigérant à reflux surmonté d'un compte-bulles contenant de l'huile de vaseline, d'un thermomètre et d'une ampoule à brome, on introduit 35 g de N-(cyanométhyl) 2-méthoxy 4-amino 5-chloro benzamide et 26 g de 1-éthylamino 2-amino propane préalablement séché et on chauffe aux environs de 130° la suspension obtenue. On ajoute alors 10 gouttes de sulfure de carbone. Il se produit aussitôt un fort dégagement d'ammoniac. En même temps, le milieu se fluidifie.

Au bout de 45 minutes, le dégagement s'arrête et la suspension initiale s'est transformée en une épaisse solution marron dans laquelle apparaît ensuite un précipité. On laisse refroidir alors le milieu réactionnel jusqu'à 30° et on le reprend à l'éther.

Le produit obtenu est essoré, lavé à l'éther et séché à l'air.

Poids obtenu = 49 g    Théorie = 47 g

Le spectre R.M.N. indique la présence d'impuretés (entre autres diamide) et d'environ 70% du composé attendu.

Ces 49 g de produit sont dissous à l'ébullition dans 15 cc de 2-éthoxy éthanol. La solution obtenue est filtrée avec du noir. Le produit recristallise très rapidement. Il est essoré, lavé avec du 2-éthoxy éthanol puis avec de l'éther et séché à l'air puis à 40°.

Poids obtenu = 25 g    Rendement de recristallisation = 51%

Ces 25 g de produit sont recristallisé dans 60 cc de 2-éthoxy éthanol. Le proudit obtenu après essorage, lavage et séchage a été recristallisé dans 26 cc de 2-éthoxy éthanol.

Poids obtenu = 10 g    Rendement de recristallisation = 40%
F = 219—221°C    Rendement des recristallisations = 20,5%
Rendement total = 20,5%

Exemple X

Nitrate de n-[(2-imidazolin-2-yl) methyl] 2-methoxy 4-amino 5-chloro benzamide

Stade 1: N-[(2-imidazolin-2-yl) methyl] 2-methoxy 4-amino 5-chloro benzamide

Dans un ballon de 500 cc muni d'un agitateur étanche, d'un réfrigérant à reflux surmonté d'un compte-bulles contenant de l'huile de vaseline et d'un thermomètre, on introduit 120 g de N-(cyanométhyl) 2-méthoxy 4-amino 5-chloro benzamide et 60 g d'éthylène diamine et on chauffe au bain d'huile l'épaisse suspension obtenue.

Après 15 minutes de chauffage, la température du bain est de 140°. On observe alors un dégagement gazeux qui s'accentue rapidement. En même temps, le milieu se fluidifie pour donner finalement une solution marron foncé.

Le mélange réactionnel est maintenu à 140° jusqu'à la fin du dégagement gazeux, soit 1 heure.

Après refroidissement, on reprend ce mélange par du chloroforme sec et on agite pendant 30 minutes. Le produit qui a cristallisé est essoré, lavé avec du chloroforme, séché à l'air puis à l'étuve.

Poids obtenu = 135 g    Rendement = 95%

Stade 2: Nitrate de n-[(2-imidzolin-2-yl) methyl] 2-methoxy 4-amino 5-chloro benzamide

80 g de base finement pulvérisés sont mis en supension dans 120 cc d'eau et 27,5 cc d'acide nitrique (d = 1,33) sont ajoutés en une seule portion. La base se dissout, puis immédiatement le nitrate cristallise. Il est refroidi, essoré, lavé à l'eau glacé jusqu'à neutralité des eaux de lavage et séché à 50°.

Poids obtenu = 35,5 g    Rendement = 36%

Ces 35,5 g sont redissous à chaud dans 175 cc de 2-méthoxy éthanol. La solution bouillante est filtrée avec du noir. La recristallisation est très rapide. Après refroidissement, le produit recristallisé est essoré, lavé avec du 2-méthoxy éthanol et séché à 40°.

Poids obtenu = 26 g    Rendement de recristallisation = 73%

Ces 26 g de base sont recristallisés dans 180 cc de 2-méthoxy éthanol. La recristallisation est toujours très rapide.

Après refroidissement, essorage et séchage, on récupère:

Poids obtenu = 21 g Rendement de recristallisation = 81%

Ces 21 g de produit sont recristallisés dans 125 cc de 2-méthoxy éthanol.

Poids obtenu = 18 g Rendement de recristallisation = 85%
P.M. ($HClO_4$ $\frac{N}{10}$) = 351,5 Rendement des recristallisations = 50%
F = 229—232°C Rendement total = 18%

La R.M.N. du produit est compatible avec la structure attendue.

Exemple XI

N-[(1-ethyl 4-dimethyl 2-imidazolin 2-yl) methyl] 2-methoxy 4-amino 5-chloro benzamide

Dans un ballon de 500 cc muni d'un agitateur étanche, d'un réfrigérant à reflux surmonté d'un compte-bulles contenant de l'huile de vaseline, d'un thermomètre et d'un ampoule à brome, on introduit 78 g de N-(cyanométhyl) 2-méthoxy 4-amino 5-chloro benzamide, 79 g de (2-amino 2-méthylpropyl) éthylamine et 5 gouttes de sulfure de carbone. On chauffe au bain d'huile la suspension obtenue.

A 130° on ajoute 5 gouttes de sulfure de carbone supplémentaires. Le milieu se fluidifie légèrement et l'ammoniac se dégage lentement. On maintient le chauffage à 130° pendant 6 heures trente jusqu'à obtention d'un épaisse solution limpide.

Cette solution est alors refroidie et reprise par 320 cc de chloroforme. Une partie se dissout, l'autre cristallise. Les cristaux sont essorés, lavés au chloroforme et séchés. On récupère ainsi 21 g de nitrile initial.

La solution chloroformique est filtrée avec du noir et concetrée sous vide jusqu'à poids constant. L'huile résiduelle est aussitôt reprise à l'éther et cristallise. Les cristaux sont essorés, lavés à l'éther et séchés à l'air.

Poids obtenu = 52 g Rendement = 47%
Rendement par rapport au nitrile entré en réaction = 64%

Produit beige foncé qui est recristallisé dans 150 cc de méthyléthylcétone.
Poids obtenu = 23 g Rendement de recristallisation = 44%

Ces 23 g de produit sont recristallisés dans 92 cc de méthyléthylcétone.
Poids obtenu = 20,5 g Rendement de recristallisation = 89%

Ces 20,5 g de produit sont redissous à chaud dans 205 cc de chlorure de méthylène et la solution obtenue est filtrée chaude avecv du noir. 90 cc de chlorure de méthylène sont ensuite distillés sous vide léger et la solution restante est refroidie. La base qui recristallise est essorée, lavée à l'éther, séchée à l'air puis à 40°.

Poids obtenu = 16 g Rendement de recristallisation 77%
P.M. ($HClO_4$ $\frac{N}{10}$ — potentiométrie) = 337
F = 185—186°C Rendement des recristallisations = 30%
Rendement total = 14%

Example XII

Chlorhydrate de n-[(1-ethyl 2-imidazolin-2-yl) methyl] 2-hydroxy 4-amino 5-chloro benzamide

Stade 1: Chlorure de 2-acetoxy 4-acetamino 5-chloro benzoyle

Dans un ballon de deux litres muni d'un réfrigérant à reflux, on introduit 501 g de chlorure de thionyle puis 71,5 g d'acide 2-acétoxy 4-acétamino 5-chloro benzoïque et on chauffe au bain d'eau au reflux, jusqu'à dissolution. On refroidit un peu et on ajoute 71,5 g d'acide 2-acétoxy 4-acétamino 5-chloro benzoïque. On chauffe de même au reflux pendant 1 heure, mais sans obtenir la dissolution de l'acide. On ajoute 63 g de chlorure de thionyle. L'acide se dissout alors immédiatement et à froid le chlorure d'acide formé cristallise rapidement.

On distille sous vide une partie du chlorure de thionyle et on reprend le résidu cristallisé à l'éther de pétrole.

Le chlorure d'acide obtenu est essoré et lavé rapidement à l'éther de pétrole et séché sous vide sur $P_2O_5$.

Poids obtenu = 143 g Rendement = 93%
P.M. ($Cl^-$ après minéralisation) = 144 × 2 = 288

Stade 2: N-(Cyanomethyl) 2-Acetoxy 4-acetamino 5-chloro benzamide

Dans un ballon de 3 litres muni d'un agitateur étanche, d'un réfrigérant à reflux et d'un thermomètre, on introduit 104 g de triéthylamine et 980 cc de chloroforme et on refroidit à 5° la solution obtenue. On y

ajoute alors peu à peu 51 g de chlorhydrate d'amino acétonitrile en 30 minutes, et ente 0° et 5°, 143 g de chlorure de 2-acétoxy 4-acétamino 5-chloro benzoyle finement pulvérisés. On obtient une solution foncée que l'on agite encore pendant 2 heures à 10° puis pendant 2 heures à température ambiante.

On distille ensuite le chloroforme sous vide et on finit d'enlever les dernières traces par distillation azéotropique avec de l'eau. Le chlorhydrate de triéthylamine se dissout, le nitrile formé cristallise. Il est essoré lavé abondamment à l'eau jusqu'à élimination des ions $Cl^-$ et séché à 50° en étuve ventilée.

Poids obtenu = 143 g    Rendement = 94%

F = 201°C

Stade 3: N-[(1-ethyl 2-imidazolin-2-yl) methyl] 2-hydroxy 4-acetamino 5-chloro benzamide.

Dans un ballon d'un litre muni d'au agitateur étanche, d'un réfrigérant à reflux surmonté d'un compte-bulles contenant de l'huile de vaseline, d'un thermomètre et d'une ampoule à brome, on introduit 143 g de N-(cyanométhyl) 2-acétoxy 4-acétamino 5-chloro benamide puis goutte à goutte rapidement 123 g de N-éthyl éthylène diamine. La réaction est exothermique. La température s'élève à 110° et le dégagement gazeux est important. On obtient une solution épaisse. On chauffe jusqu'à 120° puis après 25 minutes de chauffage on ajoute 5 gouttesde sulfure de carbone et on maintient la température à 120° pendant encore 20 minutes, jusqu'à ce que le dégagement gazeux s'arrête.

L'épaisse solution obtenue est refroidie légèrement et elle est reprise par environ 200 cc de chloroforme. Il se forme alors un précipité très fin que s'essore très lentement et de ce fait se lave très mal. Il est séché à l'air.

Poids obtenu = 76 g    Rendement = 43%

P.M. ($HClO_4$ $\frac{N}{10}$) = 298

Le spectre R.M.N. est compatible avec un mélange de 25% de produit 4 amino et 75% de 4 acétamino. Le poids moléculaire calculé de ce mélange est de 291.

Stade 4: Chlorhydrate de n[(1-ethyl 2-imidazolin-2-yl) methyl] 2-hydroxy 4-amio 5-chjloro benzamid

Dans un ballon d'un muni d'un réfigérant à reflux, on introduit 76 g de N[1-éthyl 2(imidazolin-2-yl) méthyl] 2-hydroxy 4-acétamino 5-chloro benzamide et 380 cc de méthanol contenant 25 g de gaz chlorhydrique. La solution obtenue est chauffée au reflux pendant 1 heure. Elle est ensuite concentrée jusqau'à un volume de 300 cc et refroidie. Le chlorhydrate qui cristallise est essoré, lavé avec du méthanol et séché à 40°.

Poids obtenu = 29 g    Rendement = 39%

Ces 29 g de chlrorhydrate sont redissous à chaud dans 145 cc de méthanol.

La solution bouillante est filtrée avec du noir. Maise le produit recristallise très rapidment et en partie sur le filtre. Les jus sont cependant refroidis et le chlorhydrate qui recristallise est essoré, lavé avec du méthanol et séché à 40°.

Poids obtenu = 7 g

P.M. ($AgNO_3$ $\frac{N}{10}$) = 336

Spectre R.M.N. conforme mais présence d'une petite impureté.

Le contenu du filtre est redissous à chaud dans les jus de recristallisation et la solution obtenue est concentrée, filtrée et refroidie.

Après essorage, lavage et séchage, on récupère 8 g de produit impur. Ces 8 g sont recristallisés aussitôt dans 56 cc de méthanol.

Poids obtenu = 2,5 g

P.M. ($AgNO_3$ $\frac{N}{10}$) = 338

Le spectre R.M.N. est conforme mais il reste encore une légère trace d'impureté.

Poids obtenu = 9,5 g    Rendement de recristallisation = 33%

Deux autres recristallisations dans le méthanol sont encore nécessaires pour obtenir un produit pur. Finalement on récupère:

Poids obtenu = 6 g    Rendement des recristallisations = 21%

P.M. ($AgNO_3$ $\frac{N}{10}$) = 339    Rendement total = 8%

F = 270°C environ

Le spectre R.M.N. est compatible avec la structure du produit attendu.

Exemple XIII

N-[1-(cyclohexen-1-yl-methyl) 2-(imidazolin-2-yl) methyl] 2-methoxy 4-amino 5-chloro benzamide

Dans un ballon de 500 cc mini d'un agitateur étanche, d'un réfrigérant à reflux surmonté d'un compte-bulles contenant de l'huile de vaseline, d'un thermomètre et d'une ampoule à brome, on introduit 38 g de N-(cyanométhyl) 2-méthoxy 4-amino 5-chloro benzamide et 54 g de N-(cyclohexèn-1-yl-méthyl) éthylènediamine et on chauffe au bain d'huile à 115°—120°. On ajoute alors 5 gouttes de sulfure de carbone et on continue de chauffer jusqu'à ce que le dégagement d'ammoniac commence, soit 130°. On maintient alors le chauffage à 135°—140° pendant toute la durée du dégagement gazeux (65 minutes) puis on ajoute de nouveau 3 gouttes de sulfure de carbone pour terminer la réaction.

La suspension intiale se fluidifie peu à peu pour donner finalement une solution marron.

La réaction terminée, on a refroidit jusqu'à 50°. Le produit qui commence à cristalliser est repris par un mélange de 50 cc d'éther et 25 cc d'acétone.

Il est ensuit essoré, lavé à l'éther et séché à l'air.

Poids obtenu = 42 g      Rendement = 70%

F = 158°C

Ces 42 g sont recristallisés dans 65 cc d'éthanol absolu. Après refroidissement, le produit recristallisé est essoré, lavé avec un mélange froid d'éthanol-eau (50/50) et séché à 40°.

Poids obtenu: 1er jet = 32,5 g      F = 154°C

2eme jet = 4,5 g      F = 156°C produit jaune pâle

Poids total obtenu = 37 g      Rendement de recristallization = 88%

Ces 37 g sont redissous à chaud dans 148 cc d'éthanol absolu. La solution bouillante est filtrée avec du noir, 111 cc d'éthanol sont éliminés en distillant sous vide sans dépasser 30°. Le résidu est repris par 37 cc d'eau glacée.

Le produit qui cristallise est essoré, lavé à l'eau glacé et séché à 40° en étuve ventilée.

Poids obtenu = 33 g      Rendement de recristallisation = 89%

F = 158°C

32 g sont dissous dans 96 cc de chloroforme. La solution est filtrée avec du noir et le solvant est aussitôt éliminé en distillant sous vide léger.

La pâte restante est alors reprise par 85 cc de méthyléthylcétone. Le produit qui cristallise est essoré, lavé avec de la méthyléthylcétone et séché à 40°.

Poids obtenu = 23,5 g      Rendement de recristallisation = 74%

Finalement 21 g de produit sont dissous dans 84 cc de chloroforme. La solution est filtrée avec du noir et le chloroforme est éliminé par distillation sous vide léger jusqu'à poids constant. Le solide restant, une fois pulvérisé est transvasé sur un büchner, lavé à l'éther et séché. Il est alors repris à l'eau, essoré, lavé à l'eau pour éliminer toutes traces de solvant et séché de nouveau à 40° en étuve ventilée.

Poids obtenu = 17 g      Rendement de recristallisation = 81%

P.M. ($HClO_4$ $\frac{N}{10}$ — potentiométrie) = 375

F = 152—153°C      Rendement des recristallisations = 47%

Rendement total = 33%

Exemple XIV

N-[1-(1-(1-ethyl 2-imidazolin 2-yl) ethyl] 2-methoxy 4-amino 5-chloro benzamide

Stade 1: N-(1-cyano ethyl) 2-methoxy 4-amino 5-chloro benzamide

Dans un ballon de deux litres muni d'un agitateur étanche, d'un réfrigérant à reflux, d'un thermomètre et d'une ampoule à brome, on introduit 86,5 g d'acide 2-méthoxy 4-amino 5-chloro benzoïque finement pulvérisé, 550 cc de chloroforme et 43,5 g de triéthylamine et on chauffe jusqu'à dissolution totale de l'acide. On refroidit à 0° et on coule goutte à goutte 46,5 g de chloroformiate d'éthyle en maintenant la température entre 0° et 5° par refroidissement dans un bain glacé. L'addition terminée on agite pendant 1 heure entre 5° et 10° et on ajoute goutte à goutte une solution de 33 g de 2-aminopropionitrile dans 99 cc de chloroforme. On laisse ensuite remonter la température à 20° puis on chauffe 2 heures à 50°.

On ajoute alors de l'eau à la solution obtenue et on entraîne la totalité du chloroforme. Après refroidissement, on alcalinise le milieu pour redissoudre éventuellement de l'acide n'ayant pas réagi, puis on essore le précipité qui s'est formé, on le lave à l'eau jusqu'à élimination des oins $Cl^-$ et on le séche à 50°.

Poids obtenu = 80 g Rendement = 73,5%

Ces 80 g de produit sont recristallisés dans 320 cc de 2-éthoxy éthanol.
Poids obtenu = 43 g Rendement de recristallisation = 54%
F = 220°C Rendement total = 39,5%

Stade 2: N-[1-(ethyl 2-imidazolin- 2-yl) ethyl] 2-methoxy 4-amino 5-chloro benzamide

Dans un ballon de 250 cc muni d'un agitateur étanche, d'un réfrigérant à reflux surmonté d'un compte-bulles contenant de l'huile de vaseline, d'un thermomètre et d'une ampoule à brome, on introduit 43 g de N-(1-cyanoéthyl) 2-méthoxy 4-amino 5-chlor benzamide finement pulvérisés et 30 g de N-éthyl éthylènediamine et on chauffe au bain d'huile la suspension obtenue jusqu'à 110°. On ajoute alors 10 gouttes de sulfure de carbone. Il se forme immédiatement des fumées blanches, l'ammoniac se dégage et le milieu se fluidifie peu à peu pour donner finalement une solution épaisse.

Le chauffage est maintenu jusqu'à fin du dégagement gazeux, soit 1 heure. Après refroidissement le produit cristallise. Il est repris à l'éther, essoré, lavé à l'éther, séché à l'air puis à 50°.
Poids obtenu = 50 g Rendement = 91%
F = 162°C

Ces 50 g de produit sont redissous à chaud dans 200 cc d'acétonitrile. Après refroidissement, le produit cristallisé très rapidement. Il est essoré, lavé à l'acétonitrile et séché à 50°.
Poids obtenu = 42,5 g Rendement de recristallisation = 85%

Les 42,5 g de produit redissous dans environ 180 cc de chloroforme. La solution est filtrée avec du noir et concentrée sous vide jusqu'à élimination totale du chloroforme. Le résidu est repris à l'éther. Le produit qui recristallise est essoré, lavé à l'éther et séché à l'air à 50°.
Poids obtenu = 39 g Rendement de purification = 91%
F = 180°

P.M. ($H_2SO_4$ $\frac{N}{10}$ — potentiométrie) = 327 Rendement des purification = 77%

Rendement total = 70%

## Exemple XV

N-[1-ethyl 4,5-dimethyl 2-(imidazolin-2-yl) methyl] 2-methoxy 4-amino 5-chlore benzamide

Dans un ballon de 500 cc muni d'un agitateur étanche, d'un réfrigérant à reflux surmonté d'un compte-bulles contenant de l'huile de vaseline, d'un thermomètre et d'un ampoule à brome, on introduit 39 g de N-(cyanométhyl) 2-méthoxy 4-amino 5-chloro benzamide finement pulvérisés et 38 g de N-éthyl 2,3-butane diamine et on chauffe au bain d'huile à 130°, l'épaisse suspension obtenue. On ajoute alors 6 gouttes de sulfure de carbone. Immédiatement le dégagement gazeux commence et le milieu se fluidifie peu à peu. Après une heure de chauffage, on ajoute 1 goutte de sulfure de carbone puis encore 1 goutte 1 heure après: à ce moment là, la suspension initiale s'est transformée en une solution marron, épaisse. On continue de chauffer jusqu'à la fin du dégagement gazeux, soit encore pendant 3 heures. On refroidit alors le mélange réactionnel et on obtient un produit à moitié cristallisé qui est repris à l'éther, essoré, lavé à l'éther et séché à l'air puis sous vide dans un dessicateur.
Poids obtenu = 56 g Théorie: 55 g

Ces 56 g de produit sont redissous dans 160 cc de chloroforme séché. La solution obtenue est filtrée avec du noir puis le chloroforme est distillé en terminant sous vide jusqu'à poids constant. Le résidu, repris à l'éther, cristallise lentement. Les cristaux obtenus sont essorés, lavés à l'éther puis séchés à l'air.
Poids obtenu = 56 g
F = environ 130°C.

Le produit est redissous dans 250 cc d'eau et 10 cc d'acide chlorhydrique concentré. La solution obtenue est filtrée avec du noir puis refroidie dans un bain glace-sel. Elle est ensuite transvasée dans une ampoule à décanter. 250 cc de chloroforme froid sont ajoutés puis 24 cc d'ammoniaque à 20% (virage au rose pâle de la phénolphtaléine). La base qui précipite passe immédiatement dans le chloroforme. Après décantation de la phase organique, la solution aqueuse est extraite de nouveau deux fois avec du chloroform. Les phases chloroformiques réunies sont séchées sur sulfate de sodium puis sur tamis moléculaire et filtrées avec du noir. Le chloroforme est distillé en terminant sous vide jusqu'à poids constant. Le résidu repris à l'éther cristallise immédiatement. Les cristaux obtenus sont essorés, lavés à l'éther et séchés à l'air.
Poids obtenu = 31 g Rendement = 56%
F = environ 144°C

Ces 31 g de base sont redissous dans 200 cc d'acétone bouillante. On ajoute ensuite une solution de 3,35 g de gaz chlorhydrique dans 20 cc d'acétone.

Le chlorhydrate formé précipite immédiatement sous forme d'une pâte qui cristallise en refroidissant. Le produit obtenu est essoré, lavé à l'acétone et séché.

Poids obtenu = 35 g

Le spectre RMN est compatible avec la structure attendue, mais avec une demie mole d'eau. Rendement en produit hydràté: 100%

Ces 35 g de chlorhydràte sont redissous dans 300 cc d'eau. La solution obtenue est filtrée trois fois avec du noir, puis alcalinisée par de l'ammoniaque à 20%.

On récupère ainsi:

Poids obtenu = 22 g    Rendement de la purification = 71%
F = 152—154°C    Rendement total = 40%

Les composés selon l'invention ont fait l'objet d'une étude toxicologique qui a permis de déteminer les doses léthales 50 ($DL_{50}$) chez la souris mâle, les composés étant administrés par voie intraveineuse.

Le tableau (I) rassemble les valeurs de ces ($DL_{50}$).

TABLEAU (I)

| Exemple | $DL_{50}$ en mg/kg (base) voie IV |
|---|---|
| 1 | 27,1—27,2 |
| 2 | 31,8 |
| 3 | 47,8—48,5 |
| 4 | 28—27,4 |
| 5 | 25,5—26,1 |
| 6 | 25,5—24,4 |
| 7 | 23,4—24,4 |
| 8 | 45,1—39,9 |
| 9 | 31,3—29 |
| 10 | 38,4—41,9 |
| 11 | 30,4 |
| 12 | 59,3 |
| 13 | 61,6 |
| 14 | 14,6 |
| METOCLOPRAMIDE | 37,9 |

D'autre part, une étude pharmacologique a pour objet d'évaleur la puissance de l'effet gastromoteur des composés. La méthode appliquée consistait à mesurer chez le rat la quantité évacuée d'un repas type 60 minutes après son administration, l'animal recevant le composé à étudier par voie intrapéritonéale 30 minutes avant ce repas, selon la technique décrite par DROPPLEMAN et col. J. Pharm. Meth. 1980, 4, 227—230, à trois doses différentes (1, 3, et 9 mg/kg).

Les résultants apparaissent das le tableau (II), l'activité des composés selon l'invention étant comparée à celle du Métoclopramide, pris comme composé de référence.

TABLEAU (II)

Vidange Gastrique

| Composé | dose en mg/kg (IP) (base) | poids de repas restant dans l'estomac m + es m(mg) | % de variation |
|---|---|---|---|
| 1 | | 1816 ± 47 | |
| | 1 | 1690 ± 43 | + 7 |
| | 3 | 1346 ± 26 | |
| | 9 | 1202 ± 47 | + 34 |
| 2 | — | 1854 ± 87 | |
| | 1 | 1709 ± 60 | + 8 |
| | 3 | 1461 ± 66 | + 21 |
| | 9 | 1169 ± 103 | + 37 |
| 4 | — | 1765 ± 118 | |
| | 1 | 1865 ± 95 | − 6 |
| | 3 | 1648 ± 115 | + 7 |
| | 9 | 1482 ± 128 | + 16 |
| 5 | — | 1879 ± 122 | |
| | 1 | 1716 ± 72 | + 9 |
| | 3 | 1639 ± 107 | + 13 |
| | 9 | 1350 ± 122 | + 28 |
| 6 | — | 1849 ± 54 | |
| | 1 | 1543 ± 73 | + 17 |
| | 3 | 1312 ± 43 | + 29 |
| | 9 | 1033 ± 48 | + 44 |
| 7 | — | 1891 ± 87 | |
| | 1 | 1619 ± 68 | + 14 |
| | 3 | 1566 ± 79 | + 17 |
| | 9 | 1566 ± 60 | + 18 |
| 8 | — | 2061 ± 74 | |
| | 1 | 1764 ± 99 | + 14 |
| | 3 | 1525 ± 111 | + 26 |
| | 9 | 1325 ± 84 | + 36 |
| 9 | — | 2125 ± 122 | |
| | 1 | 1635 ± 71 | + 23 |
| | 3 | 1432 ± 46 | + 33 |
| | 9 | 1355 ± 71 | + 36 |
| 10 | — | 1680 ± 86 | |
| | 1 | 1502 ± 130 | + 11 |
| | 3 | 1259 ± 71 | + 25 |
| | 9 | 1413 ± 55 | + 16 |
| 14 | — | 1817 ± 98 | |
| | 1 | 1654 ± 77 | + 9 |
| | 3 | 1453 ± 69 | + 20 |
| | 9 | 1317 ± 141 | + 28 |
| METOCLO PRAMIDE | — | 2230 ± 65 | |
| | 1 | 1645 ± 74 | + 26 |
| | 3 | 1342 ± 48 | + 40 |
| | 9 | 1232 ± 28 | + 45 |

Une autre étude pharmacologique a eu pour but de mesurer les effets antidopaminergiques centraux des composés de l'invention, en évaluant la puissance de leur effet antagoniste à l'égard des mouvements stéréotypés induits par l'apomorphine à deux doses différentes (1,25 mg/kg/IV et 0,5 mg/kg/SC) chez le rat.

Le tableau (III) indique que les composés n'exercent qu'un antagonisme négligeable, alors que, étudié dans les mêmes conditions, le Métoclopramide a révélé un pouvoir inhibiteur marqué des stéréotypies.

Tableau (III)

Stéréotypies à l'apomorphine chez le rat

| Exemple | voie S.C. | voie I.P. |
|---|---|---|
| 1 | inactif à 200 mg/kg | inactif à 50mg/kg |
| 3 | inactif à 200 mg/kg | effet inhibiteur de 19% à 64 mg/kg |
| 4 | inactif à 100 mg/kg | effet inhibiteur de 26% à 32 mg/kg |
| 5 | inactif à 200 mg/kg | effet inhibiteur de 8% à 32 mg/kg |
| 6 | inactif à 200 mg/kg | effet inhibiteur de 20% à 32 mg/kg |
| 7 | inactif à 200 mg/kg | inactif à 64 mg/kg |
| 8 | inactif à 200 mg/kg | effet inhibiteur de 8% à 64 mg/kg |
| 9 | inactif à 100 mg/kg | effet inhibiteur de 5% à 64 mg/kg |
| 10 | inactif à 100 mg/kg | effet inhibiteur de 4% à 64 mg/kg |
| 11 | inactif à 200 mg/kg | inactif à 64 mg/kg |
| 12 | inactif à 200 mg/kg | effet inhibiteur de 2% à 50 mg/kg |
| 13 | inactif à 200 mg/kg | effet inhibiteur de 18% à 64 mg/kg |
| 14 | inactif à 80 mg/kg | inactif à 40 mg/kg |
| MTC | $DI_{50}$ = 15—15,2 mg/kg | $DI_{50}$ = 2—2,6 mg/kg |

Une autre démonstration d'un effet central des composés de l'invention, différent de celui du Metoclopramide, a été faite par l'évaluation du pouvoir cataleptigène des composés, qui s'est avérée pratiquement nulle, comme l'indique la tableau (IV):

TABLEAU (IV)

| Exemple | Activité cataleptique par voie sous-cutanée |
|---|---|
| 1 | inactif à 200 mg/kg |
| 2 | inactif à 200 mg/kg |
| 3 | inactif à 200 mg/kg |
| 4 | inactif à 200 mg/kg |
| 5 | inactif à 200 mg/kg |
| 6 | inactif à 100 mg/kg |
| 7 | inactif à 200 mg/kg |
| 8 | inactif à 200 mg/kg |
| 9 | inactif à 200 mg/kg |
| 10 | inactif à 200 mg/kg |
| 11 | inactif à 200 mg/kg |
| 12 | inactif à 200 mg/kg |
| 13 | inactif à 200 mg/kg |
| 14 | inactif à 80 mg/kg |
| Métocl. | $DE_{50}$ SC + 30 − 38 mg/kg |

L'absence ou la quasi absence d'effets secondaires permet donc d'utiliser les composés de l'invention pour leurs effets gastromoteurs, avec une sécurité accrue, dans le domaine thérapeutique.

**Revendications pour les Etats contractants: BE CH DE FR GB IT LI LU NL SE**

1. Dérivés de benzamide et leurs sels pharmacologiquement acceptables, répondant à la formule générale (I):

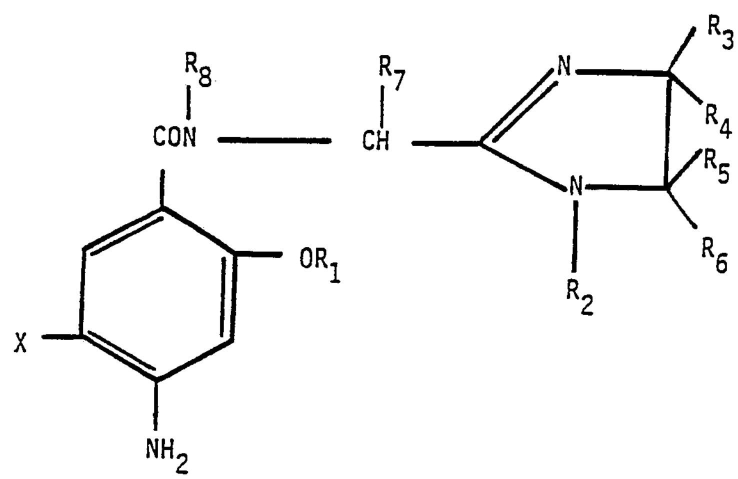

(I)

dans laquelle:

$R_1$ représente un radical méthyle ou allyle ou un atome d'hydrogène .

$R_2$ représente un radical méthyle, éthyle, allyle, benzyle, cyclopropylméthyle ou cyclohéxénylméthyle ou un atome d'hydrogène.

$R_3$, $R_4$, $R_5$, $R_6$, $R_7$, $R_8$ représentent un radical méthyle ou un atome d'hydrogène.
X représente un atome de chlore ou de brome.

2. Selon la revendication 1, le N-[(1-éthyl 2-imidazolin 2-yl) méthyl] 2-méthoxy 4-amino 5-chloro benzamide.

3. Selon la revendication 1, le N-[(1-allyl 2-imidazolin 2-yl) méthyl] 2-méthoxy 4-amino 5-chloro benzamide.

4. Selon la revendication 1, le N-[(1-éthyl 2-imidazolin 2-yl) méthyl] 2-allyloxy 4-amino 5-chloro benzamide.

5. Selon la revendication 1, le N-[(1-éthyl 2-imidazolin 2-yl) méthyl] 2-méthoxy 4-amino 5-chloro benzamide.

6. Selon la revendication 1, le méthane sulfonate de N-méthyl, N-[(1-éthyl 2-imidazolin 2-yl) méthyl] 2-méthoxy 4-amino 5-chloro benzamide.

7. Selon la revendication 1, le chlorhydrate de N-[(1-méthyl 2-imidazolin 2-yl) méthyl] 2-méthoxy 4-amino 5-chloro benzamide.

8. Selon la revendication 1, le N-[(1-éthyl 4-méthyl 2-imidazolin 2-yl) méthyl] 2-méthoxy 4-amino 5-chloro benzamide.

9. Selon la revendication 1, le N-[(1-éthyl 2-imidazolin 2-yl) éthyl] 2-méthoxy 4-amino 5-chloro benzamide.

10. Procédé de préparation des composés de formule (I) selon l'une quelconque des revendications 1 à 9 qui consiste à faire réagir un acide de formule générale (II):

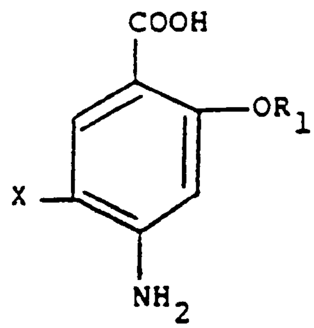

(II)

dans laquelle $R_1$ et X ont les mémes significations que précédemment, ou l'un de ses dérivés réactifs, avec un nitrile de formule genérale (III):

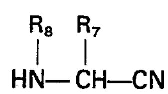

(III)

dans lequel $R_7$ et $R_8$ ont les mêmes significations que précédemment, pour obtenir un dérivé de cyanométhylbenzamide substitué de formule générale (IV):

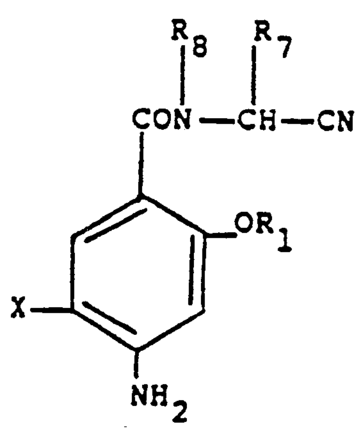

(IV)

que réagir avec une diamine substituée pour donner le composé de formule (I) attendu.

11. Selon la revendication 10, procédé de préparation des composés de formule (I), dans lequel l'acide de formule (II) réagit avec le nitrile de formule (III), sous la forme d'un ester mixte de formule générale (V):

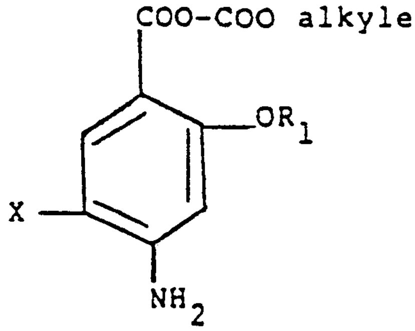

(V)

pour donner le dérivé de cyanométhyl benzamide intermédiaire (IV).

12. Selon la revendication 10, procédé de préparation des composés de formule (I), dans lequel l'acide de formule (II) réaqit avec le nitrile de formule (III) sous la forme d'un chlorure d'acide de formule générale (VI):

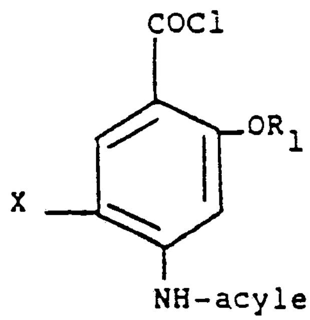

(VI)

pour donner le dérive cyanométhyl benzamide intermédiaire (IV).

13. En tant que nouveaux intermédiaires de synthèse, selon le procédé de l'une quelconque des revendications 10 à 12, les nitriles de formule générale (IV):

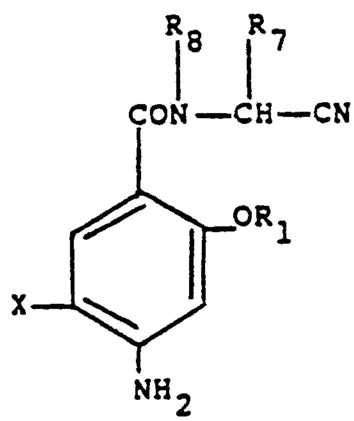

(IV)

dans laquelle $R_1$, $R_7$, $R_8$ et X ont les mêmes significations que précédemment.

14. Composés de formule (I) selon l'une quelconque des revendications 1 à 9, utilisés pour préparer des médicaments actifs, en particulier, comme gastromoteurs.

15. Composition pharmaceutique comprenant un composé de formule (I) selon l'une quelconque des revendications 1 à 9 comme principe actif associé à un excipient pharmaceutiquement acceptable.

16. Procédé de préparation d'une composition pharmaceutique qui consiste à associer à un excipient physiologiquement acceptable, l'un ou plusieurs des composés selon l'une quelconque des revendications 1 à 9.

**Revendications pour l'Autriche**

1. Procédé de préparation de dérivés de benzamide de formule (I):

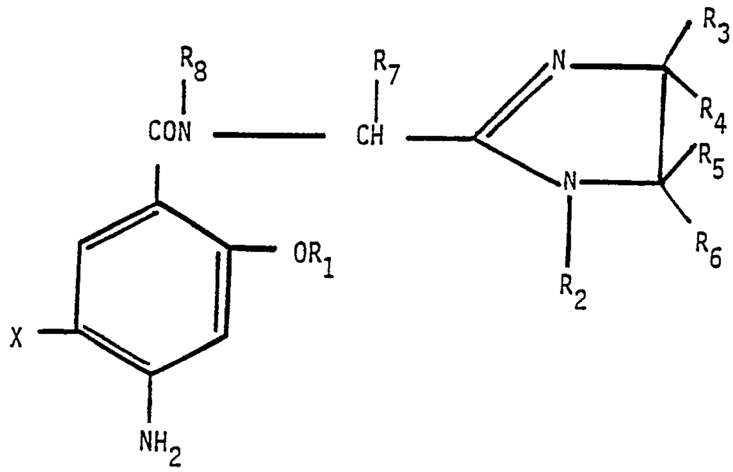

(I)

dans laquelle:

$R_1$ représente un radical méthyle ou allyle ou un atome d'hydrogène.

$R_2$ représente un radical méthyle, éthyle, allyle, benzyle, cyclopropylméthyle ou cyclohéxénylméthyle ou un atome d'hydrogène

$R_3$, $R_4$, $R_5$, $R_6$, $R_7$ et $R_8$ représentent un radical méthyle ou un atome d'hydrogène,

X représente un atome de chlore ou de brome et leurs sels pharmacologiquement acceptables, qui consiste à faire réagir un acide de formule (II):

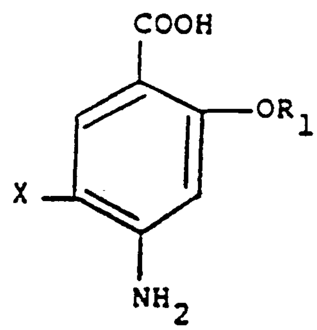

(II)

dans laquelle $R_1$ et X ont les mêmes significations que précédemment, ou l'un de ses dérivés réactifs, avec un nitrile de formule (III):

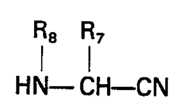

(III)

dans laquelle $R_7$ et $R_8$ ont les mêmes significations que précédemment, pour obtenir un dérivé cyanométhylbenzamide substitué de formule (IV):

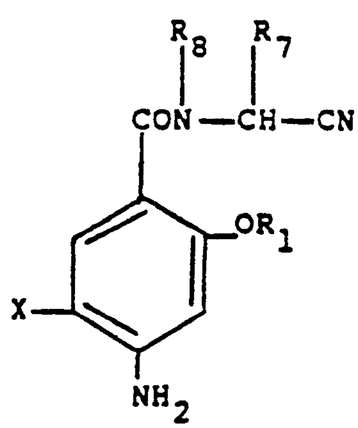

(IV)

que réagira avec une diamine substituée pour donner le composé de formule (I) attendu.

2. Selon la revendication 1, procédé de préparation des composés de formule (I), dans lequel l'acide de formule (II) réagit avec le nitrile de formule (III), , sous la forme d'un ester mixte de formule générale (V):

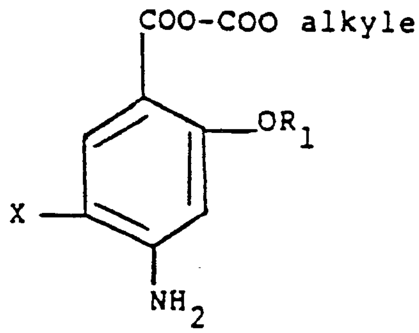

(V)

pour donner le dérivé de cyanométhyl benzamide intermédiaire (IV).

3. Selon la revendication 1, procédé de préparation des composés de formule (I), dans lequel l'acide de formule (II) réaqit avec le nitrile de formule (III) sous la forme d'un chlorure d'acide de formule générale (VI):

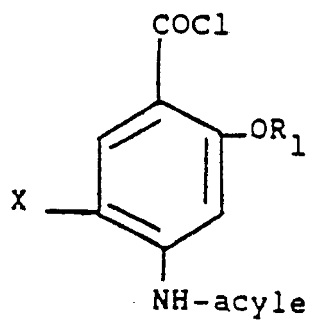

(VI)

pour donner le dérive cyanométhyl benzamide intermédiaire (IV).

**Patentansprüche für die Vertragsstaaten: BE CH DE FR GB IT LI LU NL SE**

1. Benzamidderivate und ihre pharmakoligisch verträglichen Salze gemäß der allgemeinem Formel (I)

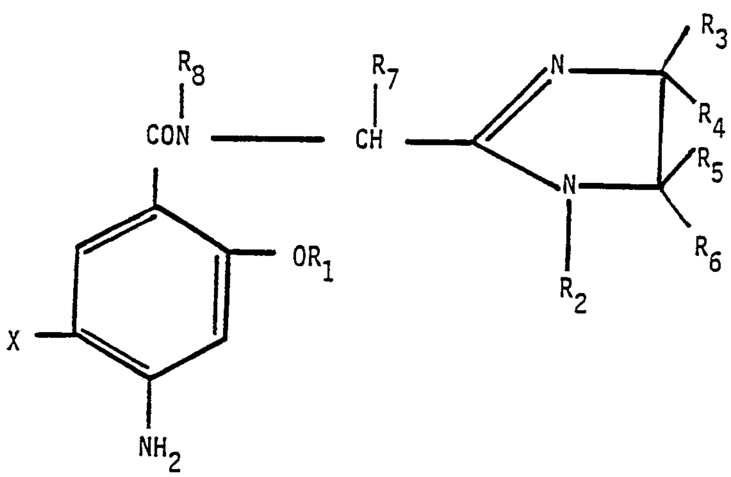

(I)

in der

$R_1$ einen Methyl- oder Allylrest oder ein Wasserstoffatom,

$R_2$ einen Methyl-, Ethyl, Allyl-, Benzyl-, Cyclopropylmethyl- oder Cyclohexenylmethylrest oder ein Wasserstoffatom,

$R_3$, $R_4$, $R_5$, $R_6$, $R_7$, $R_8$ einen Methylrest oder ein Wasserstoffatom, und
X ein Chlor- oder Bromatom bedeuten.

2. N-[(1-Ethyl-2-imidazolin-2-yl)methyl]-2-methoxy-4-amino-5-chlorbenzamid gemäß Anspruch 1.

3. N-[(1-Allyl-2-imidazolin-2-yl)methyl]-2-methoxy-4-amino-5-chlorbenzamid gemäß Anspruch 1.

4. N-[(1-Ethyl-2-imidazolin-2-yl)methyl]-2-allyloxy-4-amino-5-chlorbenzamid gemäß Anspruch 1.

5. N-[(1-Ethyl-2-imidazolin-2-yl)methyl]-2-methoxy-4-amino-5-brombenzamid gemäß Anspruch 1.

6. N-Methyl-N-[(1-ethyl-2-imidazolin-2-yl)methyl]-2-methoxy-4-amino-5-chlorbenzamid-methansulfonat gemäß Anspruch 1.

7 N-[(1-Methyl-2-imidazolin-2-yl)methyl]-2-methoxy-4-amino-5-chlorbenzamid-chlorhydrat gemäß Anspruch 1.

8. N-[(1-Ethyl-4-methyl-2-imidazolin-2-yl)methyl]-2-methoxy-4-amino-5-chlorbenzamid gemäß Anspruch 1.

9. N-[1-(1-Ethyl-2-imidazolin-2-yl)ethyl]-2-methoxy-4-amino-5-chlorbenzamid gemäß Anspruch 1.

10. Verfahren zur Herstellung der Verbindungen der Formel (I) gemäß einem der Ansprüche 1 bis 9, dadurch gekennzeichnet, daß man eine Säure der allgemeinen Formel (II)

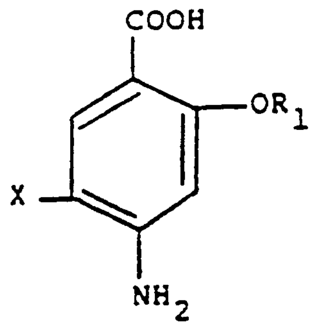

(II)

in der $R_1$ und X wie oben definiert sind, oder ein reaktives Derivat derselben mit einem Nitril der allgemeinen Formel (III)

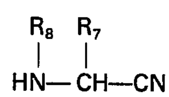

(III)

in der $R_7$ und $R_8$ wie oben definiert sind, zu einem substituierten Cyanmethylbenzamidderivat der allgemeinen Formel (IV)

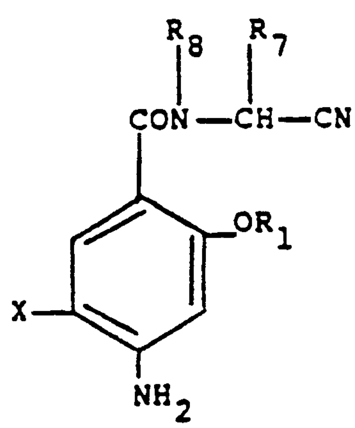

(IV)

unsetzt, das mit einem geeigneten substituierten Diamin zu der erwarteten Verbindung der Formel (I) reagiert.

11. Verfahren nach Anspruch 10 zur Herstellung der Verbindung der Formel (I), bei dem die Säure der Formel (II) mit dem Nitril der Formel (III) in der Form eines gemischten Esters der allgemeinen Formel (V)

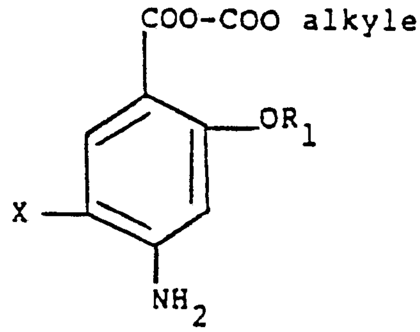

(V)

zur Bildung des Cyanmethylbenzamid-Derivats (IV) als Zwischenprodukt reagiert.

12. Verfahren nach Anspruch 10 zur Herstellung der Verbindungen der Formel (I), bei dem die Säure der Formel (II) mit dem Nitril der Formel (III) in der Forme eines Säurechlorids der allgemeinen Formel (VI)

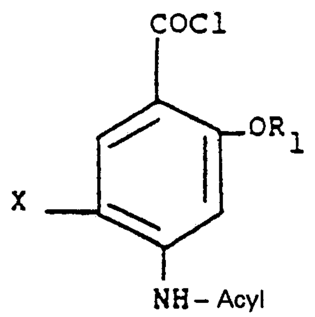

(VI)

zu dem Cyanmethylbenzamid-Derivat (IV) als Zwischen produkt reagiert.

13. Nitrile der allgemeinen Formel (IV)

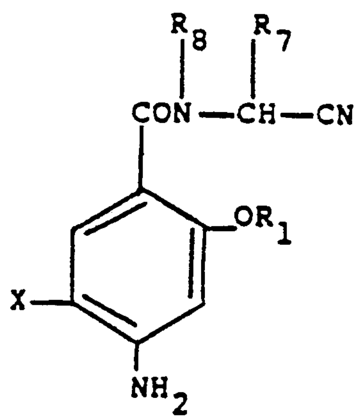

(IV)

in der $R_1$, $R_7$, $R_8$ und X wie oben definiert sind, als neue Zwischenprodukte gemäß dem Verfahren eines der Ansprüche 10 bis 12.

14. Verbindungen der Formel (I) gemäß einem der Ansprüche 1 bis 9 zur Verwendung bei der Herstellung von wirksamen Medikamenten, insbesondere Gastromotoren.

15. Pharmazeutische Zusammensetzung, enthaltend eine Verbindung der Formel (I) nach einem der Ansprüche 1 bis 9 als Wirkstoff zusammen mit einem pharmazeutisch verträglichen Exzipienten.

16. Verfahren zur Herstellung einer pharmazeutischen Zusammensetzung, dadurch gekennzeichnet, daß man einen physiologisch verträglichen Exzipienten und eine oder mehrere der Verbindungen nach einem der Ansprüche 1 bis 9 zusammengibt.

**Patentansprüche (für Österreich)**

1. Verfahren zur Herstellung von Benzamidderivaten gemäß der allgemeinen Formel (I)

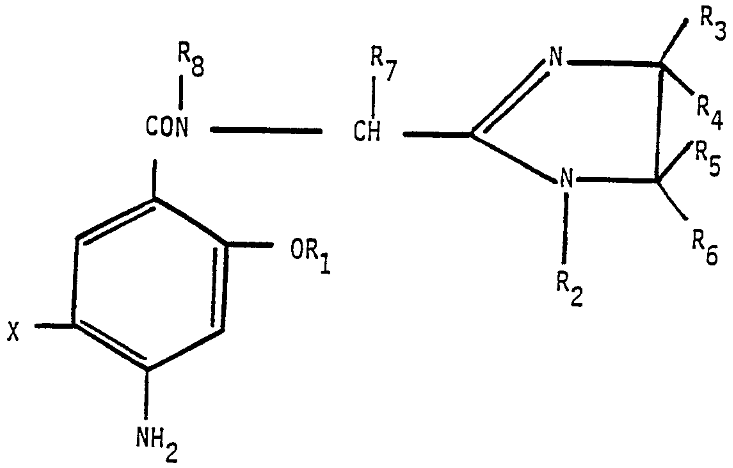

(I)

in der

$R_1$ einen Methyl- oder Allylrest oder ein Wasserstoffatom,

$R_2$ einen Methyl-, Ethyl, Allyl-, Benzyl-, Cyclopropylmethyl- oder Cyclohexenylmethylrest oder ein Wasserstoffatom,

$R_3$, $R_4$, $R_5$, $R_6$, $R_7$, $R_8$ einen Methylrest oder ein Wasserstoffatom, und

X ein Chlor- oder Bromatom bedeuten, und ihren pharmakologisch verträglichen Salzen, dadurch gekennzeichnet, daß man eine Säure der allgemeinen Formel (II)

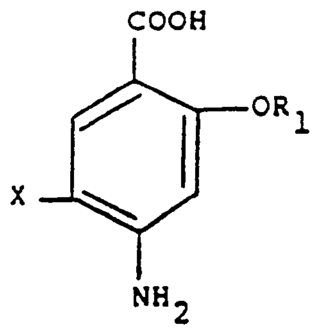

(II)

in der $R_1$ und X wie oben definiert sind, oder ein reaktives Derivat derselben mit einem Nitril der allgemeinen Formel (III)

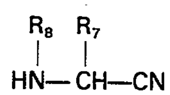

(III)

in der $R_7$ und $R_8$ wie oben definiert sind, zu einem substituierten Cyanmethylbenzamidderivat der allgemeinen Formel (IV)

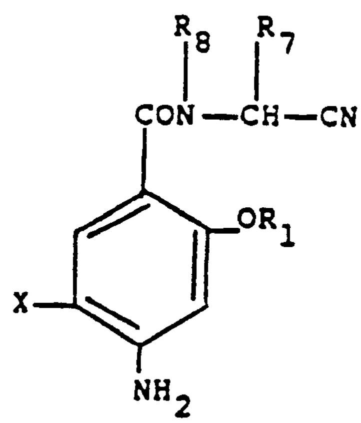

(IV)

unsetzt, das mit einem geeigneten substituierten Diamin zu der erwarteten Verbindung der Formel (I) reagiert.

2. Verfahren nach Anspruch 1 zur Herstellung der Verbindung der Formel (I), bei dem die Säure der Formel (II) mit dem Nitril der Formel (III) in der Form eines gemischten Esters der allgemeinen Formel (V)

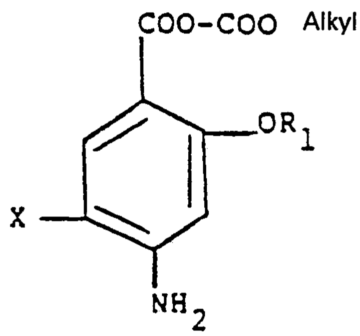

(V)

zur Bildung des Cyanmethylbenzamid-Derivats (IV) als Zwischenprodukt reagiert.

3. Verfahren nach Anspruch 1 zur Herstellung der Verbindungen der Formel (I), bei dem die Säure der Formel (II) mit dem Nitril der Formel (III) in der Forme eines Säurechlorids der allgemeinen Formel (VI)

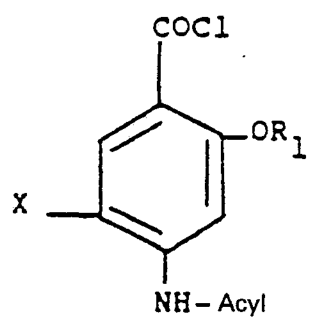

(VI)

zu dem Cyanmethylbenzamid-Derivat (IV) als Zwischenprodukt reagiert.

**Claims for the Contracting States: BE CH DE FR GB IT LI LU NL SE**

1. Derivatives of benzamide and pharmacologically acceptable salts thereof corresponding to the following general formula (I):

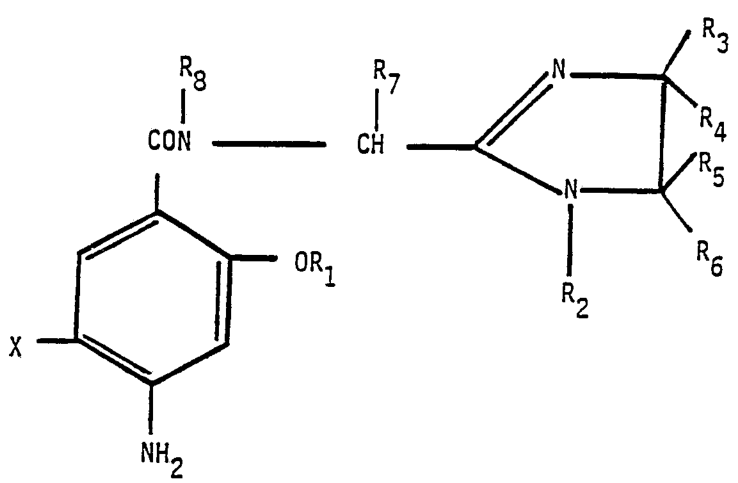

(I)

in which:

$R_1$ represents a methyl or allyl radical or a hydrogen atom,

$R_2$ represents a methyl, ethyl, allyl, benzyl, cyclopropylmethyl or cyclohexenylmethyl radical or a hydrogen atom,

$R_3$, $R_4$, $R_5$, $R_6$, $R_7$ and $R_8$ represent a methyl radical or a hydrogen atom,

X represents a chlorine or bromine atom.

2. According to claim 1, N-[(1-ethyl 2-imidazolin-2-yl) methyl] 2-methoxy 4-amino 5-chloro benzamide.

3. According to claim 1, N-[(1-allyl 2-imidazolin-2-yl) methyl] 2-methoxy 4-amino 5-chloro benzamide.

4. According to claim 1, N-[(1-ethyl 2-imidazolin-2-yl) methyl] 2-allyloxy 4-amino 5-chloro benzamide.

5. According to claim 1, N-[(1-ethyl 2-imidazolin-2-yl) methyl] 2-methoxy 4-amino 5-bromo benzamide.

6. According to claim 1, N-methyl, N-[(1-ethyl 2-imidazolin-2-yl)] methyl] 2-methoxy 4-amino 5-chloro benzamide methane sulphonate.

7. According to claim 1, N-[(1-methyl 2-imidazolin-2-yl) methyl] 2-methoxy 4-amino 5-chloro benzamide hydrochloride.

8. According to claim 1, N-[(1-ethyl 4-methyl 2-imidazolin-2-yl) methyl] 2-methoxy 4-amino 5-chloro benzamide.

9. According to claim 1, N-[(1-ethyl 2-imidazolin-2-yl) ethyl] 2-methoxy 4-amino 5-chloro benzamide.

10. A process for the preparation of the compounds of formula (I) according to any one of claims 1 to 9, which comprises reacting an acid having the following general formula (II):

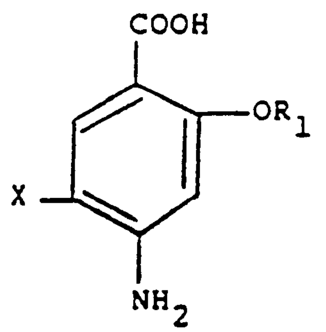

(II)

in which $R_1$ and X bear the same meanings as defined above, or one of the reactive derivatives thereof, with a nitrile having the following general formula (III):

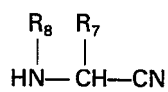

(III)

in which $R_7$ and $R_8$ have the same meanings as defined above, to produce a substituted cyanomethylbenzamide derivative having the following general formula (IV):

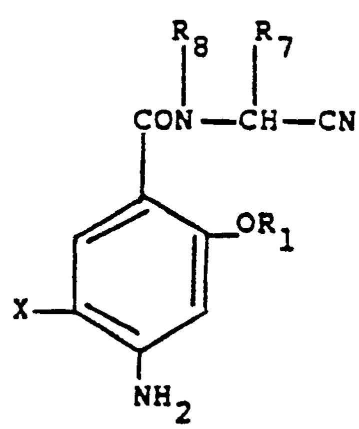

(IV)

which will react with a substituted diamine to give the expected compound of formula (I).

11. According to claim 10, process for preparation of the compounds of formula (I) in which the acid of formula (II) reacts with the nitrile of formula (III) in the form of a mixed ester of general formula (V):

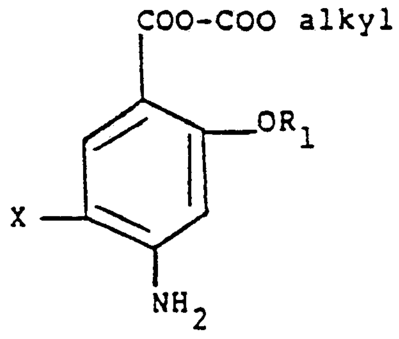

(V)

to give the intermediate cyanomethyl benzamide derivative (IV).

12. According to claim 10, process for the preparation of the compounds of formula (I) in which the acid of formula (II) reacts with the nitrile of formula (III) in the form of an acid chloride of the general formula (VI):

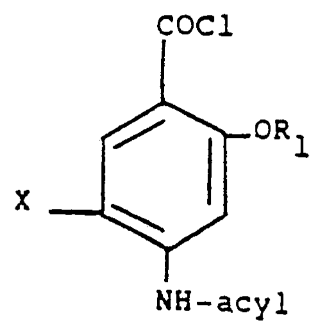

(VI)

to give the intermediate cyanomethyl benzamide derivative (IV).

13. As novel synthesis intermediates, according to the process of any one of claims 10 to 12, the nitriles of general formula (IV):

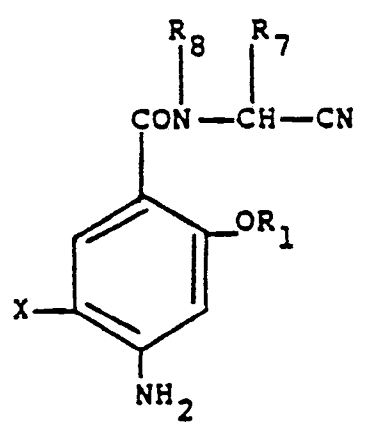

(IV)

in which $R_1$, $R_7$, $R_8$ and X are of the same meanings as defined above.

14. Compounds of formula (I) according to any one of claims 1 to 9 used to prepare medicaments, in particular as gastromotive agents.

15. Pharmaceutical composition comprising a compound of formula (I) according to any one of claims 1 to 9 as active ingredient associated with a pharmaceutically acceptable excipient.

16. A process for the preparation of a pharmaceutical composition which comprises associating with a physiologically acceptable excipient, one or more of the compounds according to any one of claims 1 to 9.

**Claims for Austria**

1. Process for preparing benzamide derivatives of general formula (I):

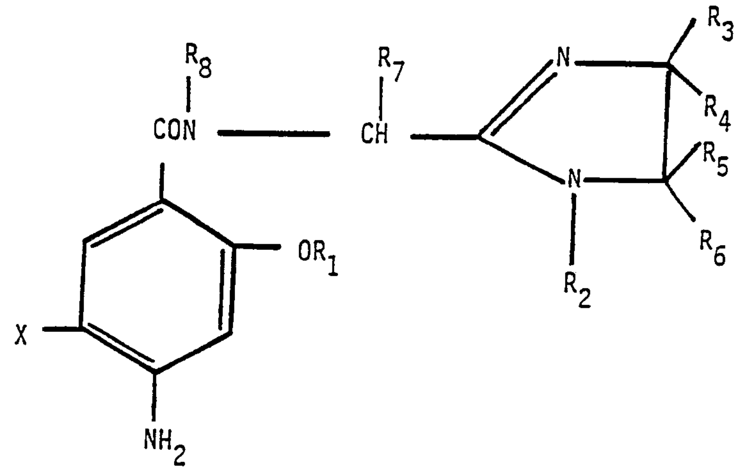

(I)

where:

$R_1$ represents a methyl or allyl group or a hydrogen atom,

$R_2$ represents a methyl, ethyl, allyl, benzyl, cyclopropylmethyl or cyclohexenylmethyl group or a hydrogen atom,

$R_3$, $R_4$, $R_5$, $R_6$, $R_7$ and $R_8$ represent a methyl group or a hydrogen atom,

X represents a chlorine or bromine atom, and their pharmacologically acceptable salts, which consists in reacting an acid of general formula (II):

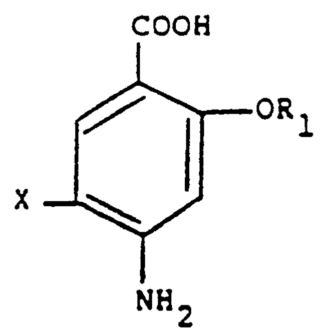

(II)

where $R_1$ and X are as defined above, or a reactive derivative thereof, with a nitrile of general formula (III):

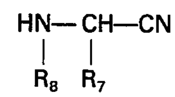

(III)

where $R_7$ and $R_8$ are as defined above, for obtaining a substituted cyanomethyl benzamide derivative of general formula (IV):

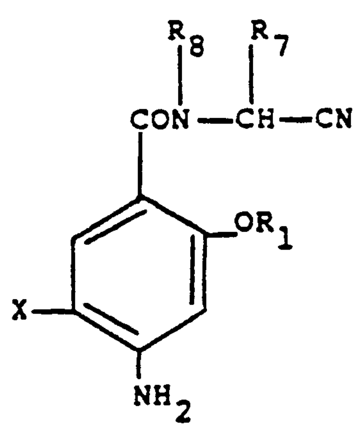

(IV)

which will react with a substituted diamine to give the expected compound of formula (I).

2. According to claim 1, process for preparing compounds of general formula (I) wherein the acid of formula (II) reacts with the nitrile of formula (III) in the form of a mixed ester of general formula (V):

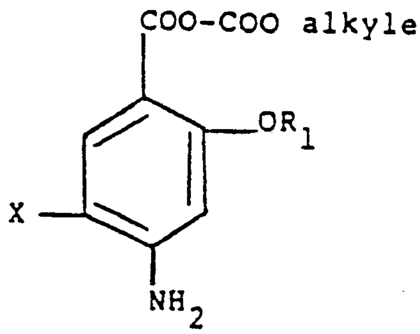

(V)

to give the intermediate cyanomethyl benzamide derivative (IV).

3. According to claim 1, process for preparing compounds of general formula (I) wherein the acid of formula (II) reacts with the nitrile of formula (III) in the form of an acid chloride of general formula (VI):

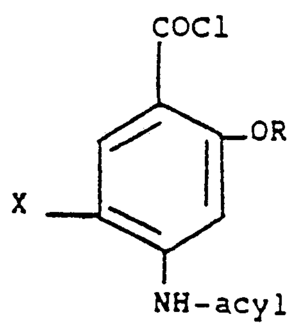

(VI)

to give the intermediate cyanomethyl benzamide derivative (IV).